# EUROPEAN PATENT SPECIFICATION

(11) **EP 2 921 174 B1**
(45) Date of publication and mention of the grant of the patent: **16.05.2018**
(21) Application number: 14193943.9
(22) Date of filing: 27.02.2008
(51) Int. Cl.: A61K 33/40, A61P 31/04, A61P 1/02

(54) **Composition and method for the prevention of oral disease**
Zusammensetzung und Verfahren zur Vermeidung von Munderkrankungen
Composition et procédé pour la prévention d'une maladie buccale

(30) Priority: 09.07.2007 US 774730
(43) Date of publication of application: 23.09.2015
(62) Divisional of application: 08743577.2
(73) Proprietor: Micropure, Inc., Scottsdale, AZ 85260 (US)
(72) Inventor: Ratcliff, James, L., Scottsdale, AZ 85255 (US); Drake, David, R., Iowan City, IA Iowa 52246 (US); Cunningham, Sally, Everett, WA 98203 (US); Renken, Elizabeth, A., Surprise, AZ 85387 (US); Young, Elena, J., Maricopa, Arizona 85138 (US)
(74) Representative: Tomkins & Co

(56) References cited:
- EP-A1- 0 613 678

## Description

### BACKGROUND OF THE INVENTION

### 1. Field of the Invention

The present invention relates to a solution for use for the prevention and treatment of oral diseases and reduction of mouth odors with the use of stabilized chlorine dioxide (ClO₂) and establishing the bactericidal and bacteriostatic properties at a concentration range of 0.1-0.8% (w/v) to significantly reduce bacterial reproduction and to act as an antimicrobial on both gram-negative anaerobic/aerobic/facultative and gram-positive anaerobic/aerobic/facultative oral bacteria.

### 2. Description of Related Art

Dental plaque has been recently defined as "a diverse community of microorganisms found on the tooth surface as a biofilm, embedded in an extracellular matrix of polymers of host and microbial origin" (Marsh, 2004). Dental plaque biofilms occur above and below the gumline, supragingival and subgingival, respectively. Acid-producing supragingival plaques are the cause of dental caries. Plaques that form on subgingival tooth surfaces and coat the epithelium lining of the gingival crevice lead to the development of periodontal infections (i.e., gingivitis and periodontitis) (Rose et al., 2004).

Supragingival dental plaque forms on teeth within hours after they are cleaned. Salivary proteins such as mucins, proline-rich proteins, statherins, histatins, and cystatins have a strong affinity for the hydroxyapatite (HAP) mineral of teeth. These proteins quickly bind to HAP of the tooth to form a thick coating called the acquired pellicle. Certain bacteria in the oral cavity selectively adhere to the pellicle, begin to divide, and form colonies. Initially, approximately 80% of the bacteria that colonize pellicle-coated tooth surfaces are facultative, gram-positive, non-motile cocci such as *Streptococcus (sanguis) sanguinis* (U.S. Patent 4,889,714). The other 20% include a variety of gram-negative bacteria such as *Veillonella* species. As the colonies grow, the environment changes due to the metabolic activities of these early colonizers and the addition of diverse groups of other bacteria to the biofilm (plaque) mass. An important environmental change in the plaque biofilm is the lowering of the local oxidation-reduction potential thus creating a low-oxygen environment that promotes the colonization and growth of anaerobic bacteria. Microorganisms in the biofilm synthesize a slime matrix or glycocalyx from abundant polysaccharides, glycoproteins, and dietary sugars (e.g., sucrose) present in the oral environment. Eventually, the plaque becomes a characteristic biofilm with a highly structured, matrix-embedded, diverse microbial population in which gene expression is severely altered (Marsh, 2005).

Bacteria in dental plaque biofilms are the major cause of several oral diseases including gingivitis, chronic and aggressive periodontitis, and necrotizing periodontal diseases. Most oral infections are surface lesions caused by normal residents of the oral microbiota and/or by exogenous pathogens that colonize the oral cavity. These infections include but are not limited to dental caries, gingivitis, periodontitis, endodontic lesions, and systemic infections that can have oral manifestations such as tuberculosis and syphilis.

Because of the association between plaque biofilms and oral diseases, there is a strong need for a formulation that will inhibit the formation of bacterial plaque by slowing the re-growth rate of plaque bacteria (gram-negative and gram-positive pathogens) and reducing the presence of organisms in mixed cultures or in a broth such as, but not limited to: *Porphyromonas gingivalis, Actinomyces odontolyticus, Actinomyces viscosus, Prevotella intermedia, Aggregatibacter (Actinobacillus) actinomycetemcomitans, Fusobacterium nucleatum, Micromonas (Peptostreptococcus) micros, Streptococcus sanguinis, Streptococcus oralis, Campylobacter rectus,* and *Enterococcus faecalis.*

Gingivitis is the inflammation of the gingival connective tissues around teeth caused by dental plaque (Rose et al., 2004). It is clinically characterized by the classical signs of inflammation such as redness (rubor), swelling (tumor), and elevated tissue temperature (calor). In addition, affected tissue bleeds when gently touched. Gingivitis is preventable by routine oral care, but if untreated can lead to a severe gum disease known as periodontitis. Periodontitis is also caused by dental plaque and is characterized by inflammation and infection of tissues that support the teeth (i.e., connective tissue and bone). If not treated it will lead to tooth loss. It is a plaque-induced inflammatory disease of the periodontium that leads to increasing probing depth, and destruction of the periodontal ligament and the adjacent supporting alveolar bone.

Halitosis, oral malodor, is caused by volatile sulfur compounds produced by bacterial metabolic degradation involved with the breakdown of organic substances in the oral cavity. Bacteria in saliva, plaque, and tongue coatings produce the volatile sulfur compounds (VSCs) that include: hydrogen sulfide (H₂S), methylmercaptan (CH₃SH), and dimethylmercaptan, which are major contributors to oral malodor (Loesche and Kazor, 2002). Oral bacteria, including gram-negative anaerobes found in subgingival plaque, produce diverse malodorous compounds including VSCs and organic acids like butyric acid, putrescine, valeric acid, and skatole (Kazor et al., 2003). More specifically, in vitro studies have shown that bacterial species associated with subgingival plaque produce large amounts of CH₃SH and H2S. These bacteria include *Fusobacterium nucleatum, Treponema denticola, Tannerella forsythia* (formerly *Bacteroides forsythus), Prevotella intermedia, Porphyromonas gingivalis, Porphyromonas endodontalis,* and *Eubacterium* species (Loesche and Kazor 2002; Kazor et al., 2003). The present invention uses stabilized chlorine dioxide as a bactericide, which leads to the reduction of oral bacteria and essentially to reduced oral malodor. Prior art compositions of oral care products of stabilized chlorine dioxide has been suggested to reduce oral malodor by acting as a deodorizing agent (U.S. Patents Nos. 4689215, 4696811, 4786492, 4788053, 4792442, 4793989, 4808389, 4818519, 4837009, 4851213, 4855135, 4886657, 4889714, 4925656, 4975285, 5200171, 5348734, 5489435,5618550).

A relationship between periodontal disease and systemic health is being established with increasing scientific evidence. Oral disease is shown to have an association with systemic diseases such as cardiovascular disease (atherosclerosis, coronary heart disease, stroke), diabetes, pre-term birth, and low birth weight (Kim and Amar, 2006).

Chronic conditions including periodontal disease, atherosclerosis, coronary heart disease, and diabetes share an exacerbated inflammatory response characteristic. The increased production of proinflammatory cytokines accompanies the exacerbated response, which occurs in response to the microbial challenge (Rose et al., 2004). However, the inflammatory response may damage healthy tissue throughout the body.

Periodontal infections stimulate the body to secrete monocyte-derived cytokines and interleukins as part of the innate immune defense system against microbial challenges. Gram-negative bacteria present in periodontal infections and dental plaque release lipopolysaccharides (endotoxin) that induce the secretion of proinflammatory cytokines. Periodontal infections may contribute directly to cardiovascular disease (e.g., atherosclerosis and thromoembolic conditions) by stimulating the immune system to produce proinflammatory cytokines (Beck et al., 1996).

Periodontal disease and its role in systemic inflammation has been shown in research identifying oral pathogenic bacteria, including *A. actinomycetemecomitans,* in atheromas in carotid arteries (Padilla et al., 2006). Evidence reported by Michaud et al. (2007) suggest that men with periodontal disease have a 63 percent higher risk of developing pancreatic cancer. The association of periodontal disease with chronic inflammation and pancreatic cancer needs more research to confirm the relationship.

Evidence indicates that there is an effect of periodontal disease on the risk of developing cardiovascular disease, which is one of the major causes of death in the U.S. Atherosclerosis is a disease of blood vessels in which endothelial cells lining the vessels are injured. Endothelial injury can be caused by a wide variety of noxious stimuli including hypertension, smoking, hyperlipidemia, toxins, viruses, immune reactions, and bacteria. In response to endothelial injury the inner wall (intima) of the vessel wall thickens. The thickened areas are called atheromas (or atheromatous plaques) that contain lipids, cholesterol, and fatty acids surrounded by a fibrous capsule. The presence of atheromatous plaque increases the risk of thrombosis or stenosis leading to decreased blood flow and myocardial infarction (heart attack).

The most important link between heart disease and periodontal disease is inflammation. Periodontal disease is one of the body's most common chronic inflammatory conditions. The inflammation occurs as a defense mechanism against bacteria that colonize the body. During the course of periodontal infections inflammatory mediators and bacteria enter the blood stream and travel to other parts of the body, such as the heart.

Briggs et al. (2006) have shown that coronary heart disease is associated with poor periodontal health in middle-aged males. This study measured serum levels of C-reactive protein (CRP), that is produced in the liver in response to a bacterial. CRP is a nonspecific marker for inflammation and is associated with an increased risk of developing cardiovascular disease. The study found that males with periodontitis have higher serum levels of CRP than individuals without periodontitis.

Periodontal infections have also been shown to be an independent risk factor for ischemic stroke (Grau et al., 2004). These investigators found that the risk for cerebral ischemia was 4.3 times higher in people with severe periodontitis compared to those with mild or no periodontitis.

*Porphyromonas gingivalis,* a major periodontal pathogen, has been shown to have higher prevalence in atherosclerotic plaques than other bacteria (Ford et al., 2005 & 2007). Other bacteria found significantly higher in arteries by Ford et al.'s research included *Fusobacterium nucleatum* and *Tannerella forsythia* (2005). These studies show that periodontal pathogens play a role in the development of atherosclerosis, as well as the fact that the immune response to multiple pathogens appears to be very complex (Ford et al., 2007). Streptococci, such as *Streptococcus sanguinis,* are common components of dental plaque from healthy sites. However, they may cause systemic problems when they enter the blood stream. Herzberg and Meyer (1998) have shown that when *S. sanguinis* is introduced into the blood stream of rabbits, the animals develop life-threatening thrombi (blood clots) in their circulatory system. The effect of periodontal disease on developing cardiovascular diseases, however, continues to be an area of investigation and more studies are needed to establish a cause-and-effect relationship between the two.

Periodontal diseases are emerging as a new risk factor for premature birth and low birth weight. It may have just as much of an impact on these complications as smoking, alcohol, drug use, and genitourinary tract infections. The relation between periodontal infections with preterm birth and low birth weight has been researched to indicate that women with periodontitis have an increased risk for adverse pregnancy outcomes (Offenbacher et al., 2001; Madianos et al., 2001). The mechanism explaining the effects of periodontitis on preterm birth suggests that microbes, including lipopolysaccharides (endotoxins), enter the uterine cavity during pregnancy and stimulate cytokine production (inflammation). The production of proinflammatory mediators lead to prostaglandin synthesis, which contracts the uterine muscles, dilates the cervix, and premature rupture of membranes (Rose et al., 2004).

Buduneli et al. (2005) found that the following subgingival bacteria, *Peptostreptococcus micros* and *Campylobacter rectus,* both play a role in increasing the risk for pre-term low birth weight. The exposure to the oral bacteria results in fetal inflammation and infection, which further increases the risk of pre-term birth (Boggess et al., 2005; Offenbacher et al., 2005). *Fusobacteriaum nucleatum,* a gram-negative oral anaerobe, was also found to be associated in adverse pregnancy outcomes by colonizing the walls of the placenta (Han et al., 2004). Further studies need to be established on the relation of periodontal disease with pre-term birth and low birth weight to develop a statistically significant association (Bassani et al., 2007).

Diabetes mellitus is a group of metabolic diseases in which hyperglycemia (elevated blood sugar) occurs due to problems with either insulin activity or its secretion. Type 1 diabetes mellitus is due to the destruction of insulin-producing beta cells in the pancreas. The beta cells can be destroyed by an environmental insult such as certain viral infections or autoimmune reactions. There is a genetic susceptibility to the disease and it primarily occurs in individuals of Northern European descent. Type 2 diabetes mellitus is due to an exhaustion of pancreatic beta cells. There is marked insulin resistance in peripheral tissues. Chronic hyperglycemia eventually results in failure of the pancreas to produce insulin. Obesity is an extremely important environmental factor that greatly increases the risk of developing type 2 diabetes. A bi-directional relation exists between diabetes and periodontal disease. Diabetes increases the risk of developing periodontal disease due to compromised response to bacterial infections (Garcia et al., 2001). Neutrophils (white blood cells) are functionally impaired in diabetics who are under poor metabolic control. Periodontal disease is also recognized as a risk for exacerbating diabetic conditions.

Diabetes and periodontal diseases are linked by the relation of inflammation and insulin resistance resulting from infection. The inflammation leads to increasing cytokine production, protein synthesis, and insulin resistance (Kim and Amar, 2006). Studies have suggested that diabetic subjects have an exaggerated response when exposed to bacteria compared to those without diabetes (Nishimura et al., 2007).

The metabolic control of diabetes is impaired in patients with untreated periodontal infections. Lim et al. (2007) investigated the relationship between glycaemic control and periodontal health and found that poor glycaemic was strongly associated with the presence of periodontal disease. The prevention of periodontal disease in diabetic patients is important for reducing the risk of developing other diabetic complications such as nephropathy, myocardial infarction, and stroke (Nishimura et al., 2007). Further studies are needed to demonstrate the significance of periodontal conditions on diabetes mellitus and all other systemic conditions.

The following bacterial species commonly found in biofilms that are considered to be periodontal pathogens in susceptible people include: *Porphyromonas gingivalis, Aggregatibacter actinomycetemcomitans, Tannerella forsythia* and *Treponema denticola* (Holt and Ebersole, 2005). Coaggregation of bacteria or the cell-to-cell adherence of microorganisms is a basic characteristic or feature of dental plaque biofilms. Coaggregation of bacteria in plaque biofilms is not a random occurrence and bacteria tend to colonize teeth as groups or in complexes. For purposes of discussion, these complexes have been given color designations to reflect developmental stages of biofilm formation and the association of certain bacterial complexes with periodontal infections (Socransky et al., 1998). Four groups referred to as the blue, yellow, green, and purple complexes are composed of early colonizers of the subgingival niche. Late colonizers associated with complex microbial communities of mature subgingival biofilms are referred to the orange and red complexes (Rose et al., 2004). The yellow complex includes several streptococci such as *S. sanguinis* and *S. oralis.* The purple complex consists of *Actinomyces odontolyticus* and *Veillonella parvula.* These species are early colonizers and are among the first to attach to the surface of the tooth. They create an environment that facilitates colonization of other bacterial complexes. The green complex includes the *Capnocytophaga* species, *Campylobacter concisus, Eikenella corrodens,* and *A. actinomycetemecomitans.* The orange cluster includes *Fusobacterium* species, *Prevotella* species, *Micromonas (Peptostreptococcus) micros, Campylobacter* species, and *Eubacterium* species. The orange complex members recognize and bind to early colonizers and promote colonization of red complex species. These bacteria are associated with 'bridging' and releasing nutrients in the biofilm for other bacteria to attach and colonize. Finally, the red complex consists of three species: *Porphyromonas gingivalis, Tannerella forsythia,* and *Treponema denticola.* These are found in greater numbers at sites with severe disease, deep pockets, and advanced lesions (Rose et al., 2004). The three species are found together in plaque areas adjacent to the epithelial lining of the gingival sulcus.

Two of the well known gram-negative periodontal pathogens are *Porphyromonas gingivalis* and *Fusobacterium nucleatum. P. gingivalis* is involved with the progression of chronic periodontitis. It has been shown to possess molecular components and a structure that facilitate its adherence to host tissue and cells, such as gingival epithelial cells. Importantly, the microorganism is an intracellular pathogen since it can invade and multiply within epithelial cells (Holt and Ebersole, 2005). *P. gingivalis* has a novel mechanism of colonizing host tissues and evading the host immune responses by spreading from cell to cell without passing through the extracellular space (Yilmaz et al., 2006). *F. nucleatum* is considered an important bacterium for the development of dental plaque biofilms and has the potential of being pathogenic. It is believed to support the growth of dental plaque acting as a bridge between early and late colonization of bacteria (Bolstad et al., 1996). It is important to health to control the rate at which such bacterial species colonize the oral cavity.

The term chlorine dioxide is widely used in the industry. Those skilled in the art will and do appreciate the various forms or variations thereof which are available to perform certain intended functions and purposes. Furthermore, U.S. Patent No. 3,271,242 describes a form of stabilized chlorine dioxide and a method of making the product, which is particularly useful in carrying out the present invention.

The bactericidal properties of chlorine dioxide were known well before its first applicable use in the 1950s (Masschelein, 1979). Chlorine dioxide, as a drinking water treatment is being obtained from sodium chlorite producing a solution free of chlorine. Stabilized chlorine dioxide is an aqueous solution comprising chlorite and stabilizers. When the pH of stabilized chlorine dioxide is lowered from a neutral pH, molecular chlorine dioxide is released from the aqueous solution. This mechanism of action of stabilized chlorine dioxide has bactericidal and bacteriostatic effects on the microbial ecology of aerobic, facultative, and anaerobic pathogenic bacteria.

The present invention relates to a solution containing stabilized chlorine dioxide for use for treatment of the mouth either in a solution, for example as a mouthwash or rinse, in concentrations below approximately 0.8% (w/v) for the control of disease-causing bacteria, bacterial plaque, and oral malodor. The rinse may also be flavored with the addition of mint oils or extracts. The flavoring would not interact with stabilized chlorine dioxide or affect the stability of the formulation.

For liquids such as mouthwash, the standard unit of measurement when expressing concentration is weight-volume percentage. That is, a certain weight of component, solid, liquid, or dissolved in a solvent, is present in a certain volume of total mouthwash. Preferred concentrations of stabilized chlorine dioxide in this invention are in the range of 0.1 % to 2% (w/v).

By "topical oral care composition" or "oral composition" as used herein is meant a product which is not intentionally swallowed for purposes of systemic administration of therapeutic agents, but is retained in the oral cavity for a sufficient time to contact substantially all of the dental surfaces and/or oral mucosal tissues for purposes of oral activity.

Previous inventions contemplate the use of stabilized chloride dioxide as a bactericide for the treatment gingivitis as well as a deodorizing agent for the treatment of oral malodor (Ratcliff, US 4,689,215; Madray, US 6,231,830; Richter, US 5,738,840; Witt, US 6,350,438). There is a large amount of evidence that indicates chlorine dioxide has bactericidal properties and that the chlorine dioxide serves to attack malodorous volatile sulfur compounds in the mouth by splitting of the sulfide bonds (Lynch, 1997; Silwood, 2001).

Prior art compositions that have been used and tested have been accepted to an extent of efficacy in treating or preventing periodontitis, gingivitis, plaque accumulation and mouth odor. Prior Ratcliff patents have demonstrated the bactericidal effect of stabilized chlorine dioxide on *Streptococcus sanguinis* (U.S. Patents Nos. 4,851,213 and 4,889,714). Herein there is described a method of reducing dental plaque by altering the ecology of oral bacteria over a period of ten seconds by means of reducing the bacterium *S. sanguinis* by more than 90%. It also describes the breakdown of double bonds in the glucosyltransferases present in the oral cavity.

Concentration range between 0.005-0.5% (w/v) chlorine dioxide with about 0.02%-3.0% phosphate is recited in U.S. Patent 5,348,734 by Ratcliff. This is the highest claimed range found in the prior art. The present invention evidences that higher concentrations of the stabilized chlorine dioxide are bactericidal for oral bacteria at a faster rate and in more complex microbial environments than prior art.

The present invention focuses on bacteriostatic properties of stabilized chlorine dioxide. Present evidence shows that the effects of stabilized chlorine dioxide on bacteria significantly reduce bacterial reproduction. There is no prior art teaching an inhibiting effect on periodontal pathogens, which renders the present invention an effective agent in prevention and treatment of oral diseases and plaque development.

It is claimed in Doyle's U.S. Patent (No. 6,846,478) that oral compositions with chlorite ions in an admixture is effective in controlling bacterial conditions and diseases in the oral cavity and inhibits the spread of bacteria into the bloodstream. In addition, U.S. Patent No. 7,087,228 (Goodman) provides a composition for preventing dental caries and infective endocarditis by treating the oral cavity for bacteria including *Streptococcus mutans.* However, these inventions are not specific to stabilized chlorine dioxide as the active ingredient for prevention and treatment of oral diseases due to polymicrobial bacterial conditions in the oral cavity.

Periodontal disease results from response to the polymicrobial ecology of subgingival plaque. The diverse and complex ecology of the oral cavity leads to a more resistant response to the immune system and antimicrobial drugs. In order to investigate a more natural environment of the oral cavity with periodontal disease, it is important to develop experimental conditions that mimic the diversity and complexity of the bacterial ecology. A limited number of studies that demonstrate considerable bacteria kill and inhibition of plaque development on such microbial environments exist. The present invention takes into consideration the diverse natural ecology and investigates both the bactericidal and bacteriostatic properties in mixed microbial suspensions of many oral bacteria involved in periodontal diseases. Previous inventions and prior research involve the investigation of single bacterial suspension cultures, which do not accurately represent the natural oral ecology.

Supporting evidence for the antimicrobial properties of the present invention are observed with Botha and Molobela's study of inhibiting individual oral organisms with a chlorine dioxide concentrated tablet (2006). The study concluded that chlorine dioxide was an effective antibacterial agent when tested against the following oral pathogens: *Streptococcus mutans, Lactobacillus casei, P. gingivalis, A. actinomycetemcomitans, P. intermedia, T. (forsythensis) forsythia,* and *E. corrodens.* The susceptibilities were varied with the different organisms, but growth inhibition was indeed observed. The results also suggest that the susceptibilities of the bacterium to chlorine dioxide do not exhibit a linear antibacterial effect to increasing chlorine dioxide concentration. The present invention supports these findings and is an extension to a comprehensive approach of representing the oral ecology as a diverse environment. EP 0 613 678 (to Micropure) describes a composition based on a stabilised chlorine dioxide solution as a mouthwash or dentifrice composition.

### Summary of the Invention

The present invention provides a solution for use as set out in the claims. A solution containing stabilized chlorine dioxide is used as a mouthwash in concentrations in the range of about 0.1% to about 0.8% (w/v) for controlling disease causing bacteria, bacterial plaque and oral malodor. The bactericidal properties include kill of anaerobic/aerobic/facultative gram-negative and gram-positive oral bacteria occurring in mixed microbial communities. Not only is the mouthwash effective in killing many periodontal pathogens on exposure, it can also slow or prevent re-colonization of oral bacteria over time.

It is therefore a primary object of the present invention to provide stabilized chlorine dioxide for use as a mouthwash to reduce plaque and oral malodor production.

Another object of the present invention is to provide a mouthwash having stabilized chlorine dioxide in a concentration in the range of about 0.1% to about 0.8% (w/v for use to cause bacteriostatic and bactericidal interference with the growth of gram-negative and gram-positive periodontal pathogens present in dental plaque biofilms).

Still another object of the present invention is to provide stabilized chlorine dioxide in a mouthwash for use to kill bacteria in a mixed microbial community within the oral cavity.

Yet another object of the present invention is to provide stabilized chlorine dioxide in a mouthwash for use to kill anaerobic/aerobic/facultative gram-negative and gram-positive bacteria in the oral cavity.

A further object of the present invention is to provide a mouthwash having stabilized chlorine dioxide in a concentration in the range of about 0.1% to about 0.8% (w/v) for use for the prevention of oral disease through its bactericidal and bacteriostatic properties.

These and other objects of the present invention will become apparent to those skilled in the art as the description thereof proceeds.

### BRIEF DESCRIPTION OF THE DRAWINGS

The present invention will be described with greater specificity and clarity with reference to the drawings, in which:
Figure 1 shows the bacterial viability after one (1) minute exposures to unflavored 0.1% (w/v) concentration oral rinse at 0 hours, 17 hours, and 36 hours of incubation against a mixed bacterial suspension;
Figure 2 shows the bacterial viability after one (1) minute exposures with distilled water (control) at 0, 17 and 36 hours of incubation, which serves as the negative control for comparison to the 0.1% concentration oral rinse shown in Figure 1;
Figure 3 shows the bacterial viability after one (1) minute exposures to unflavored 0.4% (w/v) concentration oral rinse at 0, 17 and 36 hours of incubation against a mixed bacterial suspension;
Figure 4 shows the bacterial viability after one (1) minute exposures to unflavored 0.5% (w/v) concentrated oral rinse at 0, 17 and 36 hours of incubation against a mixed bacterial suspension;
Figure 5 shows the bacterial viability after one (1) minute exposures to distilled water (control) at 0, 17 and 36 hours of incubation against a mixed bacterial suspension, which serves as a comparison to the unflavored 0.5% (w/v) concentrated oral rinse shown in Figure 4;
Figure 6 shows the bacterial viability after one (1) minute exposures to flavored 0.5% (w/v) concentrated oral rinse at 0, 17 and 36 hours of incubation against a mixed bacterial suspension (Note the recurrence of a count of *F. nucleatum* at exposure 3, this could be due to bacterial resistance or a technical error);
Figure 7 shows the bacterial viability after one (1) minute exposures to distilled water (control) at 0, 17 and 36 hours of incubation against a mixed bacterial suspension, which serves as a comparison to the flavored 0.5% (w/v) concentrated oral rinse shown in Figure 6;
Figure 8 shows the bacterial viability after one (1) minute exposures to flavored 0.6% (w/v) concentrated oral rinse at 0, 17 and 36 hours of incubation against a mixed bacterial suspension;
Figure 9 shows the bacterial viability after one (1) minute exposures to unflavored 0.6% (w/v) concentrated oral rinse at 0, 17 and 36 hours of incubation against a mixed bacterial suspension comparison between the rinse and the controls (dH₂O and ETSB-YE) are also shown;
Figure 10 shows the bacterial viability after one (1) minute exposures to flavored 0.8% (w/v) concentrated oral rinse at 0, 17 and 36 hours of incubation against a mixed bacterial suspension; a comparison between the rinse and the controls (dH₂O and ETSB-YE) is also shown;
Figure 11 shows the bacterial viability after one (1) minute exposures to unflavored 0.8% (w/v) concentrated oral rinse at 0, 17 and 36 hours of incubation against a mixed bacterial suspension; a comparison between the rinse and the controls (dH₂O and ETSB-YE) is also shown; and
Figure 12 shows the growth inhibition of *Porphyromonas gingivalis* (ATCC 33277) after one (1) minute exposures to the unflavored oral rinse (T₀ Assay Concentration = 10⁸); controls included in this test are chlorhexidine, neutralizing broth, dH₂O, and Pg *(Porphyromonas gingivalis)* broth.

### DESCRIPTION OF THE PREFERRED EMBODIMENT

The present invention concerns oral care solutions including oral washes or rinses in a solution comprising of stabilized chlorine dioxide. It contemplates the use of stabilized chlorine dioxide as a bactericidal and bacteriostatic agent against microorganisms involved in oral disease such as, but not limited to, *Porphyromonas gingivalis, Actinomyces odontolyticus* and *A*. *viscosus, Prevotella intermedia, Fusobacterium nucleatum, Micromonas micros, Streptococcus sanguinis* and *S. oralis, Campylobacter rectus,* and *Enterococcus faecalis.* The mechanism for the said solution includes the determination of bactericidal activity of the stabilized chlorine dioxide against a spectrum of oral bacteria, as single bacteria and as polymicrobial communities, associated with periodontal diseases and health.

The present invention consists of a stabilized chlorine dioxide solution for use as a bactericide and/ or bacteriostatic on oral bacterial communities at a concentration range between 0.1 %-0.8% (w/v). It has an effect of killing and reducing the number of gram-negative and gram-positive bacteria at concentrations higher than known in the prior art. This effect was unanticipated because it was believed that a linear relationship in the amount of bacterial kill would be exhibited. However it was discovered that an exponential rate of bacterial kill occurred in the in vitro studies. It was assumed that at 0.2% (w/v) concentration the kill rate would have been twice that of the 0.1% (w/v) concentration, as determined in a linear relationship. This correlation was not observed in tests done as evidence of the present invention. There seems to be an exponential effect on the microorganisms as the concentration of stabilized chlorine dioxide is increased.

The present invention establishes the bactericidal kinetics of the antimicrobial characteristics of stabilized chlorine dioxide against mixed bacterial communities at a concentration range of 0.1-0.8% (w/v). The experimental approach of evaluating bacteria as a community and not as a single entity is important because oral bacteria do not occur in the mouth as single species, but as a community of complex and diverse bacterial species. Conventional testing does not test bacterial kill in plaque-like environments or in polymicrobial suspensions, as was done in the experimentation. This environment reduces the susceptibility to antimicrobials and makes it more difficult to control and prevent progression into diseased conditions.

The present invention acts as a bactericide on the following bacteria: *Porphyromonas gingivalis, Actinomyces odontolyticus* and *A. viscosus, Prevotella intermedia, Fusobacterium nucleatum, Micromonas micros, Streptococcus sanguinis* and *S*. *oralis, Campylobacter rectus,* and *Enterococcus faecalis.* It is believed to be effective on the majority of oral bacteria involved in the progression of oral diseases and periodontitis. When suspended in a polymicrobial culture of multiple species of oral bacteria, the solution kills and reduces the re-growth of the oral bacteria associated with oral diseases and opportunistic bacteria including those associated with health, more specifically both gram-negative anaerobic/aerobic/facultative and gram-positive anaerobic/aerobic/facultative oral bacteria.

The present invention proclaims the use of stabilized chlorine dioxide oral rinse to be used as a bacteriostatic treatment on oral bacteria in mixed cultures over a period of 48 hours. It contemplates the ability of stabilized chlorine dioxide as a bacteriostatic agent against oral bacteria involved in periodontal diseases. For example, it was shown in the present invention that the re-growth of *P. gingivalis* was inhibited, showing a bacteriostatic effect on this specific gram-negative periodontal pathogen. There is little or no prior research or art claiming inhibited growth of oral bacteria, such as *P. gingivalis,* after exposure to stabilized chlorine dioxide. Research suggests stabilized chlorine dioxide caused bacteriostatic effects on the bacterial cells ultimately leading to cell death. This inhibition of cellular metabolism and cell function effectively inhibits or controls the formation of bacterial plaque and malodorous volatile sulfur compounds, the main contributors to oral disease and plaque formation.

The specific mechanism in which chlorine dioxide inactivates bacteria is currently postulated and researched. Therefore, it is believed that the present invention's bacteriostatic properties are due to inhibition of protein synthesis and/or to the inability of the cell to maintain membrane permeability and inhibited metabolic processes. Due to these effects on bacteria, plaque production and progression to oral diseases can be inhibited by rinsing with a solution of the stabilized chlorine dioxide in a concentration range of 0.1% to 0.8% (w/v). The production of malodorous oral compounds (VSCs) can also be inhibited. The following mechanisms of action specify the explanations for bacterial kill by chlorine dioxide.

The specific mechanism of action of chlorine dioxide on cells has been debated for a number of years. Early research showed that chlorine dioxide's primary effect was the disruption of protein synthesis, leading to cell death (Benarde et al., 1967). Results from Benarde's studies clearly showed an abrupt inhibition on protein synthesis. Explanations of this occurrence on the cells included possible inhibition of amino acid activation, inactivation of messenger RNA (which prevents translation), and destruction of ribosomes by chlorine dioxide (which causes a loss in cell contents by leakage).

A later study, however, indicated that this might not be the case. Roller et al. studied the effects of chlorine dioxide on dehydrogenase enzymes, protein synthesis, and deoxyribonucleic acid of bacteria (Roller et al., 1986). Results showed that total dehydrogenase enzymes were inhibited completely within the first 5 seconds of reaction by chlorine dioxide and protein synthesis was partially inhibited. The dosage of chlorine dioxide used was found to be proportional to the extent of inhibition. These studies concluded that the primary effect of chlorine dioxide on cells was occurring in an area in the cell other than the dehydrogenase enzymes, protein synthesizing complex, or DNA. It was determined that inhibition of protein synthesis of cells, indeed, contributed to cell death. However, Roller et al. concluded that an impairment of the cell's functions are occurring even before protein synthesis. Chlorine dioxide was shown to not cause cell inactivation by altering or impairing the cell's DNA. An explanation or theory of the cell deaths by chlorine dioxide in this study is by a reaction with or oxidation of components related to enzyme activity of the cell (Roller et al., 1986).

A more recent study on the mechanism of action of chlorine dioxide was done by Berg et al. (1986). Berg et al. studied the effect of chlorine dioxide on membrane functions of *Escherichia coli* and found that the permeability control was impaired, leading to cell death. It was also shown that the inactivation by the chlorine dioxide does not cause a significant loss of intracellular macromolecules existing inside the cell to the surroundings. However, the membrane damage led to the loss of intracellular potassium destroying the transmembrane ionic gradient, which is understood to result in lethal inhibition of the metabolic processes and cell death. Thus, the permeability barrier of the cell was determined to be important to the sensitivity to chlorine dioxide and growth characteristics of the cell.

The present research evidence suggests stabilized chlorine dioxide causes bactericidal and bacteriostatic effects on the bacterial cells which ultimately lead to cell death. These effects can lead to control over the formation of bacterial plaque and malodorous volatile sulfur compounds, main contributors to oral diseases.

To test the bactericidal properties of stabilized chlorine dioxide on single oral bacteria species and on mixed bacterial suspensions, solutions of specified concentrations (concentration range between 0.1%-0.8% (w/v)) were tested in vitro in assays containing the bacterium. Experimentations included testing both non-flavored and flavored oral rinse solutions.

To test the bacteriostatic properties of stabilized chlorine dioxide, a solution consisting of 0.1% (w/v) concentration stabilized chlorine dioxide was tested in vitro with oral bacteria.

### Materials:

*Actinomyces viscosus* ATCC 43146
*Actinomyces odontolyticus* ATCC 17929
*Campylobacter rectus* ATCC 33238
*Enterococcus faecalis* ATCC 10741
*Fusobacterium nucleatum* ATCC 49256
*Micromonas (Peptostreptococcus) micros* ATCC 33270
*Porphyromonas gingivalis* ATCC 49417
*Prevotella intermedia* ATCC 25611
*Streptococcus sanguinis* ATCC 10556
*Streptococcus oralis* ATCC 35037

Oral rinse with flavoring agent
Oral rinse without flavoring agent
Difco™ Neutralizing Broth (∼800 mLs)
Enriched TSBYE (∼400 mLs)
Artificial Salvia+5%Tryptone+5%Yeast Extract (∼2,500 mLs) (AS+T+Y)
   0.147 g/L Calcium Chloride (CaCl₂)
   0.426 g/L Sodium Phosphate (Na₂HPO₄)
   1.68 g/L Sodium Bicarbonate (NaHCO₃)
90-50 mL Centrifuge Tubes
25 mL, 10 mL, 5mL, and 0.1 mL Pipets
Pipet-aid Pipeter
9 sterile Erlenmeyer flasks
Sterile test tubes
39-Each selective agar for a total of 312 plates (pre-reduced for 48 hrs)
Optimal growth media and selective agars (media was pre-reduced for 24 hrs):
   CR broth and CR agar-*C*. *rectus*
   BHIB broth and BHIB pH=9.6 agar*-E. faecalis*
   TSBYE invertase treated broth and MSss/MSN agar-*S*. *sanguinis* and *S. oralis* Schaedler broth and CVE agar-*F*. *nucleatum*
   Pm broth and MSCN agar-*M*. *micros*
   Pg broth and BPB+MUP agar-*P*. *gingivalis*
   BHIB broth and CFAT+MUP agar-*A*. *viscosus*
   ETSBYE-for all bacteria
Blood Agar

Determination of bactericidal activity of the stabilized chlorine dioxide rinse was performed on individual oral bacterial suspensions which are associated with gingivitis, periodontal diseases and health. The following bacteria were assayed individually in a microtiter plate assay system: *Porphyromonas gingivalis, Prevotella intermedia, Fusobacterium nucleatum, Micromonas micros, Actinomyces viscosus, Actinomyces odontolyticus, Streptococcus sanguinis, Streptococcus oralis, Campylobacter rectus* and *Enterococcus faecalis.*

The organisms used in the experiments are American Type Culture Collection (ATCC) isolates and were originally obtained from oral infections. All bacteria are permanently stored as a cryolibrary at -90°C.

Primary cultures were grown in standard, enriched media appropriate for the species in an anaerobic chamber (Coy) at 37°C for 48 hours. Cells were harvested by centrifugation and washed 2X in sterile, pre-reduced media. Cells were suspended in sterile, pre-reduced media and adjusted to standardized concentrations based on standard curves of cell concentration and absorbance via spectroscopy.

Bactericidal assays were performed in a microtiter plate setup. Assay components consisted of standardized bacterial suspensions, with starting cell concentrations of 10⁶-10⁷ CFU/mL, antimicrobial dilutions, and sterile media. A range of concentrations of rinse were used, diluted in sterile distilled water. Positive controls consisted of 0.12% chlorhexidine and negative controls were sterile distilled water (dH₂O). In a number of runs, the flavoring agent was also included in the assay.

Microtiter plates were incubated in an anaerobic chamber between assay time points. Samples were removed at different periods of time and processed to determine the number of viable cells via spiral-plating methodology using a Spiral Plate 4000 system. The spiral plate system was used to determine bacterial cell counts and the extent of antimicrobial susceptibilities. Exposure times of 1, 5 and 10 minutes were observed.

Raw microbial counts were initially transformed to log₁₀ values to normalize the data before performing statistical analysis. This is a standard practice and is widely used to evaluate numbers of bacteria in antimicrobial assays. Data were analyzed mostly by one-way analysis of variance (ANOVA). In some cases, if unequal variances were encountered, data were also analyzed via a nonparametric Kruskall-Wallis ANOVA. In the former case, if statistical significance was observed, Tukey-Kramer post-tests were run to determine differences between groups. In the latter, Dunn's multiple comparison's tests were run. Statistical significance was set at α = 0.05. Data were analyzed by Student's t-test.

A general trend among the gram-negative anaerobes was observed, these bacterium were more susceptible to the stabilized chlorine dioxide rinse at 0.1% (w/v) concentration (data not shown). A reduction in the number of viable cells following a 1-minute exposure were observed for *Fusobacterium nucleatum,* however the data were not statistically significant. Similarly, a reduction of viable cells was observed with *Porphyromonas gingivalis.* However, a complete kill of inocula was observed in experiments with a 10-minute exposure.

A slightly different susceptibility profile was observed with *Prevotella intermedia,* anaerobic gram-negative bacteria. Statistically significant decreases in numbers of viable cells were observed with both a 1 and 5 minute exposure, with complete kill occurring at 5 minutes.

Results from *Campylobacter rectus* experiments are indicate that at 1 -minute exposure to oral rinse concentrations resulted in statistically significant reductions in the numbers of viable cells in the assay. The oral rinse resulted in a >95% kill with a 1-minute exposure to *Campylobacter rectus* (anaerobic gram-negative).

In conclusion, the oral rinse has shown a strong bacterial viability effects to gram-negative anaerobic oral bacteria. Further, these bacteria were tested in polymicrobial mixes, which is associated with the development and progression of periodontal diseases and infections.

The composition of the polymicrobial mixes was designed to include organisms associated with health and disease (consisting of the same bacterium used in the individual oral bacterial suspension tests above).

The purpose of this experiment was to determine if there is a bactericidal effect on a mixed bacterial suspension when the suspension is exposed to the oral rinse at multiple times (0, 17, and 36 hours) for duration of one minute at concentrations ranging from 0.1% to 0.8% (w/v). The following methods are detailed for 0.4% (w/v) oral rinse concentration, which was modified accordingly for other concentrations tested, including 0.1%, 0.3%, 0.4%, 0.5%, 0.6%, and 0.8% (w/v) concentrations.

The testing done was similar to that performed for individual bacteria, only the oral rinse was assessed against mixed bacterial suspensions. Each of the bacterial organisms (0.1 mL for aerobes and 0.5 mL for anaerobes) were inoculated in 50 mLs of optimal growth media and were incubated for 24 hours (aerobes) and 48 hours (anaerobes). Once the incubation period was complete, 25 mLs of the bacterial stocks were centrifuged at 7500 times gravity (7500xg) for 10 minutes and re-suspended in 25 mLs of ETSBYE. Two 10⁻² dilutions were performed from these re-suspensions into ETSBYE (0.1 mL of re-suspension in 10 mLs of ETSBYE). The second of the dilutions was plated on blood agar so as to obtain a CFU/mL count of the originally grown cultures. To verify the *F. nucleatum* starting concentration for the mixed suspension, 3.5 mLs of initial culture was added to 6.5 mLs of Schaedler broth. This was then diluted 10⁻² and 10⁻⁴ and spiral plated on blood agar and CVE agar.

The following table shows the volume of each bacteria added to create 35 mL of a mixed culture suspension at 1.2x10⁸ CFU/mL. Once the mixed suspension was created, the suspension was diluted 10⁻² and 10⁻³ and spiral plated on the selective agars and blood agar respectively. A660 is the absorbance at 660nm, which determines the number of cells in suspension.

| Mixed Culture Suspensions | | |
|---|---|---|
| Bacteria | Volume Added to Achieve Mixed Culture Suspension (∼1x10⁶) | A660 |
| *E. faecalis* | 2.00 | 1.160 |
| *S. oralis* | 0.38 | 1.386 |
| *S. sanguinis* | 2.15 | 1.043 |
| *A. viscosus* | 8.70 | 0.238* |
| *C. rectus* | 1.98 | 0.502 |
| *F. nucleatum* | 12.00 | 1.030, 1.005 |
| *M. micros* | 3.50 | 0.258** |
| *P. gingivalis* | 2.80 | 1.544 |
| ETSBYE | 1.49 | |

| | | |
|---|---|---|
| **--the A660 was so low because bacteria had adhered to the bottom of the flask. The bottom of the flask was scraped with a sterile loop to allow them to go into suspension and the CFU*/*mL counts for the assay came out to be what was expected.* ***--the concentration appeared to be double via the A660, however, according to the standard curve, there was a minute difference and the experiment was therefore run as previously calculated.* | | |

The oral rinse flavored and unflavored tubes were conducted in triplicate together and the dH₂O and ETSBYE tubes (controls) were conducted in triplicate together. Each tube was prepared as follows: the bacterial suspension was added, and the centrifuge tube was vortexed for one minute. Immediately following the one minute exposure, 22 mLs were removed and added to a centrifuge tube containing 22 mLs of neutralizing broth. This centrifuge tube was then vortexed for 30 seconds. The tubes were reduced and then centrifuged at 8,000xg for ten minutes. The supernatant was removed and 44 mLs of pre-reduced AS+T+Y were added. The tubes were then centrifuged at 8,000xg for an additional ten minutes. The supernatant was again removed and 3 mLs of AS+T+Y were added. Dilutions of 10⁻² were performed and spiral plated onto the selective agars. The tubes were then incubated anaerobically in a 37°C incubator for 17 hours and the process was repeated. This process was performed at 0 hours, 17 hours, and 36 hours.

As in the testing done above, microtiter plates were incubated in an anaerobic chamber between assay time points. Samples were removed at different periods of time and processed to determine the number of viable cells via spiral-plating methodology using a Spiral Plate 4000 system.

Statistical analyses were done in the same manner as done in individual bacterial suspension studies, described above.

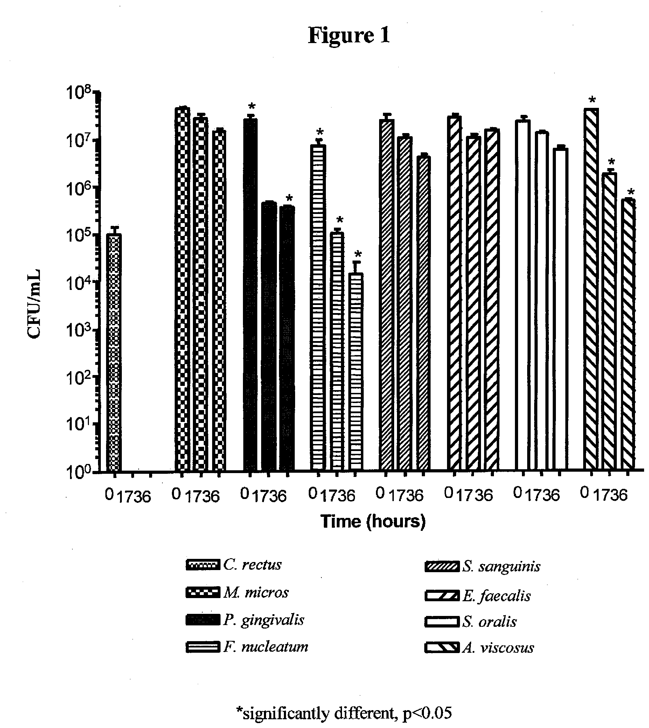

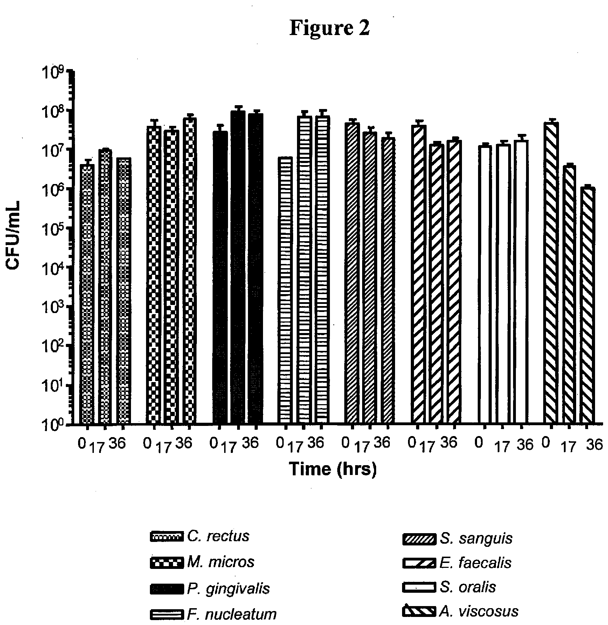

Results for the effect of the oral rinse at 0.1% (w/v) concentration against the polymicrobial suspensions are shown in Figure 1. Complete kill of *C*. *rectus,* a gram-negative anaerobe and periodontal pathogen, occurred after the second exposure (17 hours) to the oral rinse at 0.1% (w/v) concentration. There were also significant reductions in the number of viable cells of the other bacteria tested including *F. nucleatum* and *P. gingivalis..* Figure 2 shows the control of distilled water exposures, which indicates the significant effect of the oral rinse on the kill of polymicrobial bacteria when compared to Figure 1. Tests done that included a flavoring agent in the oral rinse solution, showed a complete kill of both *C*. *rectus* and *P. gingivalis* after the second exposure (data not shown).

At 0.1% (w/v) concentration oral rinse, gram-positive bacteria normally associated with oral health showed little change following the multiple exposure regimens. But higher concentrated rinses exhibited a much higher level of bactericidal activity (0.4, 0.5, 0.6 and 0.8%). Exposures of polymicrobial suspensions to the concentrated rinses resulted in complete kill of all of the gram-negative and gram-positive organisms as soon as after the second exposure.

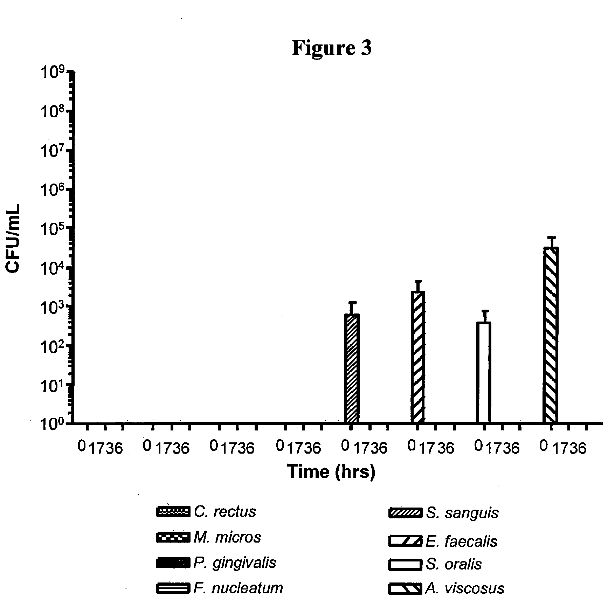

Results indicated that the anaerobic, gram-negative periodontal pathogens in polymicrobial suspensions were highly susceptible to multiple, brief exposures to the stabilized chlorine dioxide oral rinse. The 0.4% (w/v) oral rinse exhibited very strong bactericidal activity, resulting in significant reductions in levels of all bacteria within the polymicrobial suspensions, as shown in Figure 3. More specifically, only four gram-positive bacteria survived the first exposure to the oral rinse *(S. sanguinis, E. faecalis, S. oralis,* and *A. viscosus).* Importantly, gram-negative anaerobes such as *Porphyromonas gingivalis, F. nucleatum,* and *C*. *rectus* were completely eliminated after the first exposure to the oral rinse.

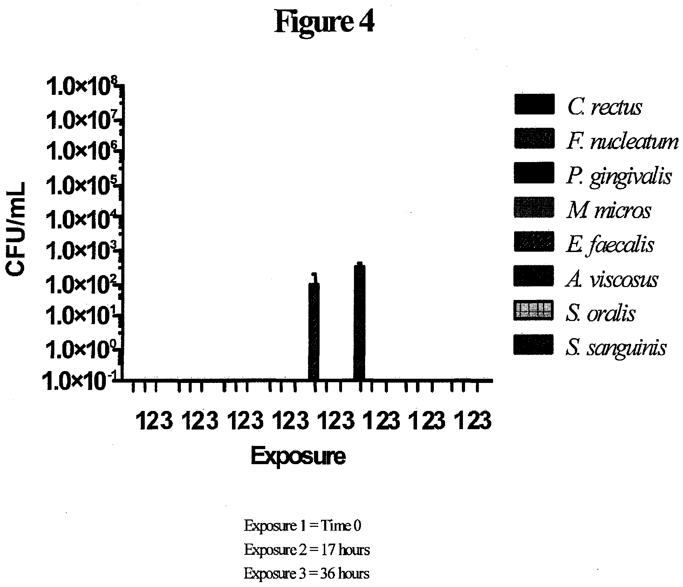

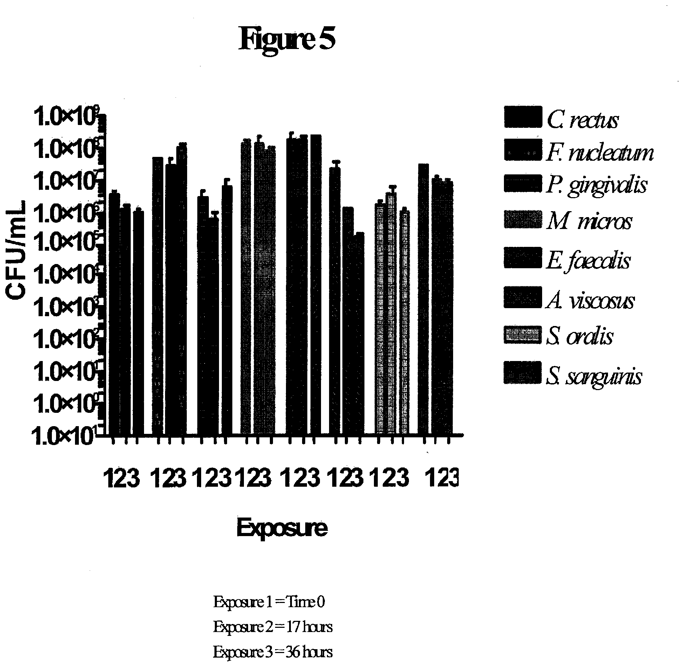

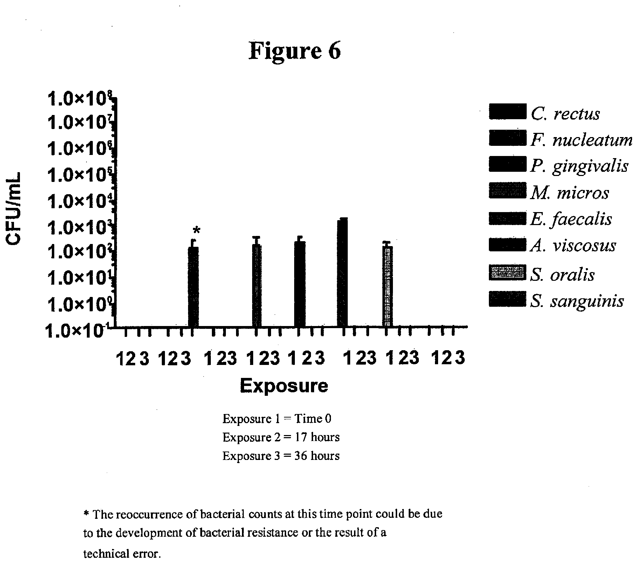

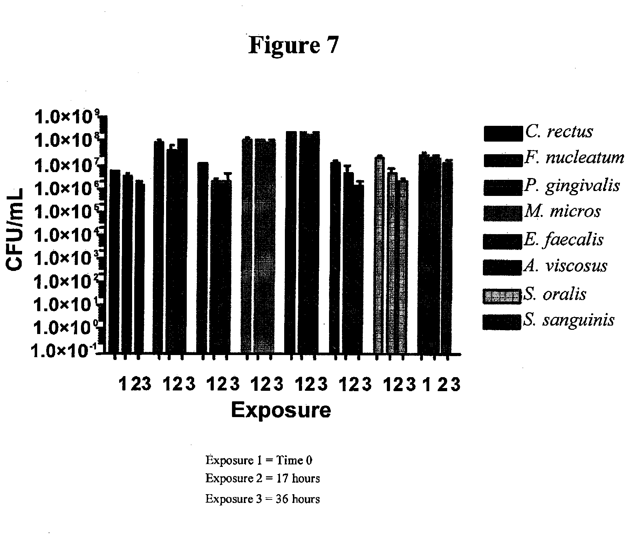

The level of bactericidal activity with the 0.3% (w/v) rinse was very similar to the 0.1% (w/v) concentration rinse (data not shown). This occurrence is believed to be an exponential effect of kill on the bacterium from the oral rinse, because there was a sudden increase in bacterial viability in response to the oral rinse in concentrations 0.4% (w/v) and above. This outcome is represented in the figures representing results from 0.4, 0.5, 0.6 and 0.8% oral rinse tests. At 0.5% concentration of stabilized chlorine dioxide, with and without the flavoring agent, results in complete kill of all bacteria in the polymicrobial suspension after multiple exposures over 36 hours as shown in Figures 4 and 6. As illustrated, in Figure 4 only two types of oral bacteria survived after the first exposure and were completely killed off at the second exposure after 17 hours; these two bacterium were *E. faecalis* (gram-positive anaerobic bacteria) and *A*. *viscosus* (gram-positive aerobic bacteria). The results obtained from the exposures to the stabilized chlorine dioxide can be compared to the controls of exposure to distilled water shown in Figures 5 and 7 to compare and observe the significant differences clearly.

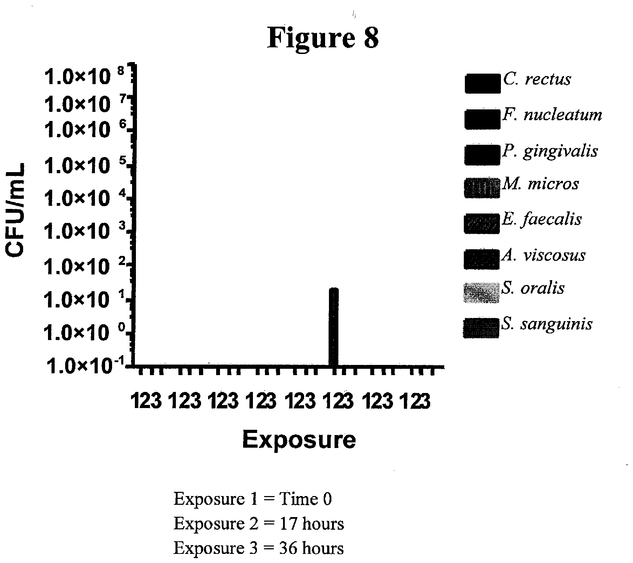

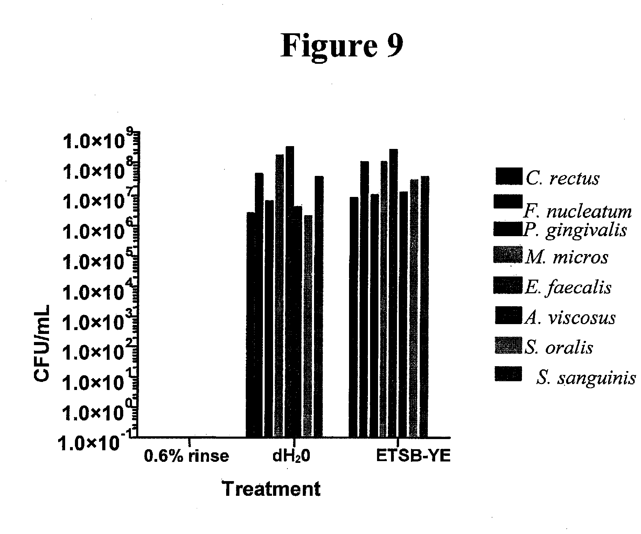

Figures 8 to 11, show the bactericidal activity of the oral rinse at concentrations 0.6% and 0.8% (w/v) with and without the flavor agent (flavored and unflavored). The 0.6% concentrated oral rinse completely killed all bacteria in the mixed bacterial suspension after a first exposure in the unflavored oral rinse. The only bacterium that survived the first exposure in the flavored oral rinse was *A. viscosus,* a gram-positive bacterium. This bacterium was, however, killed after the second exposure 17 hours later. The controls of dH₂O and ETSB-YE are also shown in part with the 0.6% oral rinse, as illustrated in Figure 9. It can be seen that there were live cells in the controls.

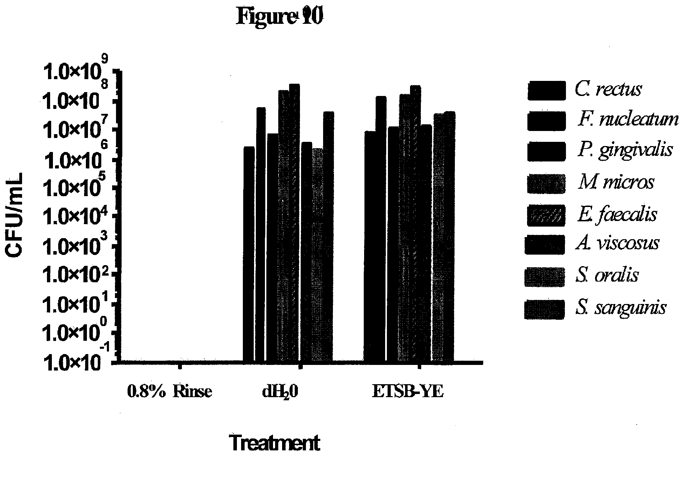

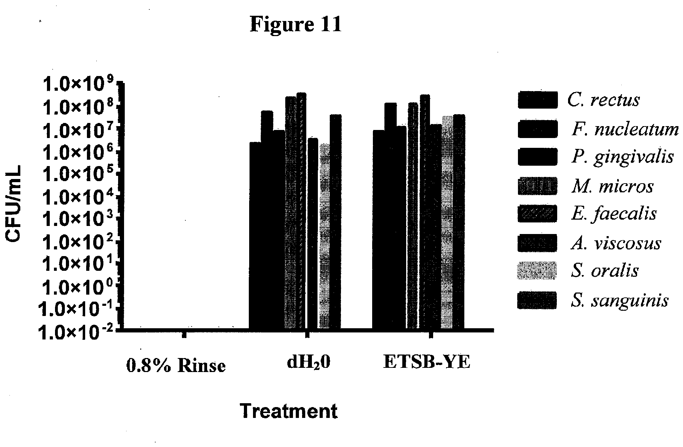

At the highest concentration tested, 0.8% (w/v), there was no sign of living bacteria after the first exposure in both the flavored and unflavored oral rinses, as shown in Figures 10 and 11, respectively. Again, the controls in both figures can be compared to the oral rinse.

In conclusion, concentrated stabilized chlorine dioxide oral rinses, as high as 0.8% (w/v) possess a high level of bactericidal activity targeting gram-negative anaerobes and gram-positive aerobes/anaerobes. The results demonstrate an increasing step function relationship of the bactericidal effect and the concentration of the oral rinse. There is a significant increase in bacterial kill in a polymicrobial suspension at 0.4% concentration and above. A linear increase does not appear to be the case due to the change in effect from the 0.1% and 0.3% concentrations to the 0.4% concentration oral rinse on the kill of oral bacteria.

The plaque re-growth experiments were performed to demonstrate the bacteriostatic effect of the stabilized chlorine dioxide rinse on oral bacteria, such as *Porphyromonas gingivalis.* Suspensions of *Porphyromonas gingivalis,* adjusted to an assay concentration of 10⁶-10⁸ CFU/mL, were exposed to the 0.1% (w/v) concentration of oral rinse for one minute. The suspensions were immediately diluted into fresh medium and then incubated at 37°C in an anaerobic chamber.

Evidence suggests the stabilized chlorine dioxide oral rinse is an effective bacteriostatic agent, which inhibits the regeneration of oral bacteria over a period of 48 hours. The bacteriostatic properties of chlorine dioxide would implicate it as an effective solution in preventing degradation of exogenous and endogenous proteinaceous substrates, oral epithelium, food debris and saliva. Most importantly, this would limit the production of dental plaque and volatile sulfur compounds.

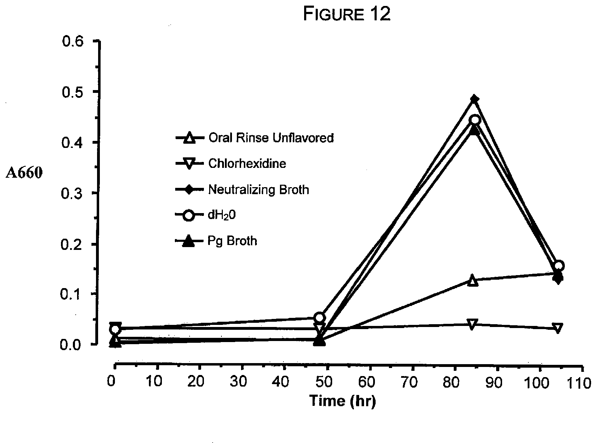

Cells exposed to distilled water or fresh medium exhibited normal growth kinetics, reaching a high absorbance by 36 hours of incubation. However, cells exposed to the oral rinse, either flavored or unflavored, were not able to grow over a 48 hour period. This effect was observed with *P. gingivalis* at 10⁶-10⁷ CFU/mL. The inhibition of growth of *P. gingivalis* is shown in Figure 12 as a result of unflavored 0.1% (w/v) concentration oral rinse. Based on the data obtained, it was observed that a brief exposure to the oral rinse has a significant bacteriostatic effect on the gram-negative periodontal pathogen, *Porphyromonas gingivalis.* It is believed to also have an effect on the other oral bacterium.

The addition of the flavor agent to the oral rinse also has the same effect of inhibiting the growth of *P. gingivalis* (data not shown). It is known that flavor agents usually have bactericidal properties and the effect was anticipated in these tests. Additionally, it can be determined that the flavoring agent has a beneficial effect when used with the stabilized chlorine dioxide oral rinse.

## Claims

1. A solution of stabilized chlorine dioxide having a concentration in the range of about 0.1% (w/v) to about 0.8% (w/v) for use to cause bacteriostatic and/or bactericidal interference with the growth of gram-negative and gram-positive periodontal pathogens present within polymicrobial mixtures in the oral cavity.

2. The solution for use as set forth in Claim 1 for creating a bacteriostatic and bactericidal interference with the growth of anaerobic and aerobic bacteria in polymicrobial mixtures associated with periodontal diseases.

3. The solution for use as set forth in Claim 1 for interfering with protein production to negatively affect the growth of gram-negative and gram-positive periodontal pathogens.

4. The solution for use as set forth in Claim 1 for interfering with the production of at least one of DNA and RNA to negatively affect the growth of gram-negative and gram-positive periodontal pathogens.

5. The solution for use as set forth in Claim 1 for affecting a bacteria cell's ability to maintain membrane permeability control.

6. The solution for use as set forth in Claim 1 for at least reducing the presence of gram-negative periodontal pathogens including any of *Porphyromonas gingivalis, Prevotella intermedia, Fusobacterium nucleatum, and Campylobacter rectus,* in polymicrobial mixtures within the oral cavity.

7. The solution for use as claimed in Claim 1 for at least reducing the presence of gram-positive periodontal bacteria in polymicrobial mixtures including any of *Actinomyces odontolyticus, Actinomyces viscosus, Streptococcus sanguinis, Streptococcus oralis, Micromonas (Peptostreptococcus) micros, and Enterococcus faecalis.*

8. The solution for use as set forth in Claim 1 for predominantly achieving bacteriostatic effects on the growth of periodontal pathogens in a polymicrobial mixtures of gram-negative and gram-positive bacteria wherein the concentration of stabilized chlorine dioxide is in the range of 0.1% (w/v) to 0.3% (w/v).

9. The solution for use as set forth in Claim 1 for use to achieve predominantly bactericidal effects on periodontal pathogens in a polymicrobial mixture of gram-negative and gram-positive bacteria wherein the concentration of stabilized chlorine dioxide is in the range of 0.4% (w/v) to 0.8% (w/v).

10. The solution for use as set forth in Claim 1 wherein the bacteriostatic and bactericidal properties are due to inhibition of protein synthesis and/or to the inability of the bacterial cell to maintain membrane permeability control, and inhibited metabolic processes.

## Patentansprüche

1. Lösung von stabilisiertem Chlordioxid mit einer Konzentration im Bereich von ungefähr 0,1% (Gew./Vol) bis ungefähr 0,8% (Gew./Vol.), zur Verwendung, um eine bakteriostatische und/oder bakterizide Beeinträchtigung des Wachstums gramnegativer und grampositiver parodontaler Erreger bzw. Pathogene zu bewirken, die in polymikrobiellen Mischungen in der Mundhöhle vorhanden sind.

2. Lösung zur Verwendung nach Anspruch 1, um eine bakteriostatische und bakterizide Beeinträchtigung des Wachstums anaerober und aerober Bakterien in polymikrobiellen Mischungen, die mit parodontalen Erkrankungen assoziiert sind, zu erzeugen

3. Lösung zur Verwendung nach Anspruch 1, um die Proteinproduktion zu beeinträchtigen, um das Wachstum von gramnegativen und grampositiven parodontalen Erregern negativ zu beeinflussen.

4. Lösung zur Verwendung nach Anspruch 1, zum Beeinträchtigen der Produktion von zumindest DNA und/oder RNA, um das Wachstum von gramnegativen und grampositiven parodontalen Erreger negativ zu beeinflussen.

5. Lösung zur Verwendung nach Anspruch 1, zur Beeinträchtigung einer Fähigkeit von Bakterienzellen, die Membranpermeabilitätskontrolle aufrechtzuerhalten.

6. Lösung zur Verwendung nach Anspruch 1, um zumindest die Anwesenheit von gramnegativen parodontalen Erregern, die irgendeinen von *Porphyromonas gingivalis, Prevotella intermedia, Fusobacterium nucleatum und Campylobacter rectus* aufweisen, in polymicrobiellen Mischungen in der Mundhöhle zu reduzieren.

7. Lösung zur Verwendung nach Anspruch 1, um zumindest die Anwesenheit von grampositiven parodontalen Bakterien in der polymikrobiellen Mischungen, zu reduzieren. die irgendeinen von *Actinomyces odontolyticus, Actinomyces viscosus, Streptococcus sanguinis, Streptococcus oralis, Micromonas (Peptostreptococcus) micros* und *Enterococcus faecalis* aufweisen.

8. Lösung zur Verwendung nach Anspruch 1, um vorwiegend bakteriostatische Effekte auf das Wachstum parodontaler Erreger in einer polmikrobiellen Mischung von gramnegativen und grampositiven Bakterien zu erreichen, wobei die Konzentration von stabilisiertem Chlordioxd im Bereich von 0,1% (Gew./Vol.) bis 0,3% (Gew./Vol.) liegt.

9. Lösung zur Verwendung nach Anspruch 1, um vorwiegend bakteriostatische Effekte auf parodontale Erreger in einer polmikrobiellen Mischung von gramnegativen und grampositiven Bakterien zu erreichen, wobei die Konzentration von stabilisiertem Chlordioxid im Bereich von 0,4% (Gew./Vol.) bis 0,8% (Gew./Vol.) liegt.

10. Lösung zur Verwendung nach Anspruch 1, wobei die bakteriostatischen und bakteriziden Eigenschaften auf Folgendem beruhen, der Inhibition bzw. Hemmung der Proteinsynthese und/oder der Unfähigkeit der Bakterienzelle, die Steuerung der Membranpermeabilität aufrechtzuerhalten und auf gehemmten Stoffwechselprozessen.

## Revendications

1. Solution de dioxyde de chlore stabilisé ayant une concentration comprise entre environ 0,1 % (poids/volume) et environ 0,8 % (poids/volume) pour servir à créer une interférence bactériostatique et/ou bactéricide avec la croissance d'éléments pathogènes parodontaux à Gram négatif et à Gram positif présents dans des mélanges polymicrobiens dans la cavité buccale.

2. Solution pour utilisation selon la revendication 1, pour créer une interférence bactériostatique et bactéricide avec la croissance de bactéries anaérobies et aérobies dans des mélanges polymicrobiens associés à des maladies parodontales.

3. Solution pour utilisation selon la revendication 1, pour interférer avec une production de protéines pour affecter négativement la croissance d'éléments pathogènes parodontaux à Gram négatif et à Gram positif.

4. Solution pour utilisation selon la revendication 1, pour interférer avec la production d'au moins l'un de l'ADN et de l'ARN pour affecter négativement la croissance d'éléments pathogènes parodontaux à Gram négatif et à Gram positif.

5. Solution pour utilisation selon la revendication 1, pour affecter la capacité de cellules bactériennes à maintenir un contrôle de perméabilité de membrane.

6. Solution pour utilisation selon la revendication 1, pour au moins réduire la présence d'éléments pathogènes parodontaux à Gram négatif comprenant l'un quelconque de *Porphyromonas gingivalis, Prevotella intermedia, Fusobacterium nucleatum,* et *Campylobacter rectus,* dans des mélanges polymicrobiens dans la cavité buccale.

7. Solution pour utilisation selon la revendication 1, pour au moins réduire la présence de bactéries parodontales à Gram positif dans des mélanges polymicrobiens comprenant l'un quelconque de *Actinomyces odontolyticus, Actinomyces viscosus, Streptococcus sanguinis, Streptococcus oralis, Micromonas (Peptostreptococcus) micros,* et *Enterococcus faecalis.*

8. Solution pour utilisation selon la revendication 1, pour obtenir de manière prédominante des effets bactériostatiques sur la croissance d'éléments pathogènes parodontaux dans un mélange polymicrobien de bactéries à Gram négatif et à Gram positif, dans laquelle la concentration en dioxyde de chlore stabilisé est comprise entre 0,1 % (poids/volume) et 0,3 % (poids/volume).

9. Solution pour utilisation selon la revendication 1, pour servir à obtenir de manière prédominante des effets bactéricides sur des éléments pathogènes parodontaux dans un mélange polymicrobien de bactéries à Gram négatif et à Gram positif, dans laquelle la concentration en dioxyde de chlore stabilisé est comprise entre 0,4 % (poids/volume) et 0,8 % (poids/volume).

10. Solution pour utilisation selon la revendication 1, dans laquelle les propriétés bactériostatiques et bactéricides sont dues à l'inhibition de la synthèse de protéines et/ou à l'incapacité de la cellule bactérienne à maintenir un contrôle de perméabilité de membrane, et à des processus métaboliques inhibés.
